# EUROPÄISCHE PATENTSCHRIFT

(11) **EP 0 470 098 B1**
(45) Veröffentlichungstag und Bekanntmachung des Hinweises auf die Patenterteilung: **07.12.1994**
(21) Anmeldenummer: 90906151.7
(22) Anmeldetag: 17.04.1990
(51) Int. Cl.: C07C 31/125, C07C 29/136

(54) **VERFAHREN ZUR KATALYTISCHEN HYDRIERUNG VON FLÜSSIGEN FETTSÄURE-METHYLESTERN**
PROCESS FOR THE CATALYTIC HYDROGENATION OF LIQUID FATTY ACID METHYL ESTERS
PROCEDE D'HYDROGENATION CATALYTIQUE D'ESTERS METHYLIQUES D'ACIDES GRAS LIQUIDES

(30) Priorität: 24.04.1989 DE 3913387
(43) Veröffentlichungstag der Anmeldung: 12.02.1992
(73) Patentinhaber: Henkel Kommanditgesellschaft auf Aktien, 40191 Düsseldorf (DE)
(72) Erfinder: FLECKENSTEIN, Theo, D-4010 Hilden (DE); GÖBEL, Gerd, D-5000 Köln 40 (DE); CARDUCK, Franz-Josef, D-5657 Haan (DE); DEMMERING, Günther, D-5650 Solingen 1 (DE); KUBERSKY, Hans-Peter, D-5650 Solingen 1 (DE)
(86) Internationale Anmeldenummer: EP9000597
(87) Internationale Veröffentlichungsnummer: WO9012775

(56) Entgegenhaltungen:
- EP-A- 0 300 346
- EP-A- 0 334 118

## Beschreibung

Die Erfindung betrifft ein Verfahren zur katalytischen Hydrierung von flüssigen gesättigten und ungesättigten Fettsäure-Methylestern im C-Kettenbereich von C₆ bis C₂₄ zur Gewinnung von gesättigten Fettalkoholen und Methanol in Gegenwart von gasförmigem Wasserstoff und Hydrierkatalysatoren bei Drücken von 50 bis 300 bar und Temperaturen von 160 bis 250 °C.

Bei derartigen, in Großanlagen durchgeführten Verfahren werden bisher Rieselbettreaktoren verwendet, die im wesentlichen Schachtreaktoren mit Durchmessern bis zu 2 m und mit Reaktorlängen zwischen 5 m und 10 m sind. Der für die Hydrierung notwendige Katalysator liegt als regellose Schüttung im Reaktor verteilt vor (Ullmann's Enzyklopädie der Technischen Chemie, 4. Auflage, Band 11, Seiten 433 und 434). Die Reaktionsführung in diesen Reaktoren erfolgt adiabatisch, d.h. bei den meist exothermen Reaktionen steigt die Temperatur längs der Katalysatorschüttung an. Da die Selektivität der meisten Katalysator-Typen aber stark temperaturabhängig ist, kann es aufgrund der Temperaturänderungen im Reaktor zu Veränderungen des Reaktionsmechanismus kommen, so daß daher meist unerwünschte Neben- oder Folgereaktionen stattfinden. Zusätzlich können Übertemperaturen zu einer irreversiblen Schädigung des Katalysators führen.

Ferner kommt es in den bekannten Schachtreaktoren trotz gleichmäßig über die Querschnittsfläche verteilter Flüssigkeitsaufgabe zu einer ungünstig breiten Verweilzeitverteilung. Dabei verringert sich der Reaktornutzungsgrad.

Es ist zwar bekannt, zur Begrenzung der Temperaturerhöhung die Gasphase im hohen Überschuß durch den Reaktor zu führen oder mehrere, hintereinandergeschaltete Reaktoren mit Zwischenkühlung zu benutzen. Diese Art der Temperaturführung ist aber für die Hydrierung von Fettsäuremethylester nicht ausreichend, da sich die unerwünschten Folgereaktionen auf diese Weise nicht ausschließen lassen und die Katalysatoren beim Überschreiten gewisser Temperaturen durch Rekristallisationsvorgänge und Strukturveränderungen Aktivitätsverluste erleiden können.

Es ist auch bekannt, katalytische Reaktionen in einem isotherm betriebenen Rohrbündelreaktor durchzuführen (Deutsche Zeitschrift Chemie-Technik, 4. Jahrgang (1975), Nr. 12, Seiten 439 bis 441). Nach dem sogenannten Bayer-Kalthydrier-Prozess werden katalytische Hydrierungen bei niedrigen Temperaturen nahezu isotherm durchgeführt, so daß der Katalysator keinen Temperaturschwankungen unterworfen ist und die Gefahr der Überhitzung nicht besteht. Bei der in dieser Literaturstelle beschriebenen Reaktion handelt es sich jedoch um eine recht unkritische Hydrierreaktion, da das Reaktionsprodukt nicht oder nicht in diesem Maße der Weiterreaktion unterliegt, so daß notfalls nicht umgesetzte Anteile im Kreis geführt werden können oder auch eine verlängerte Verweilzeit unter Hydrierbedingungen das Produkt nicht schädigt.

Der Erfindung liegt die Aufgabe zugrunde, ein Verfahren der eingangs genannten Art zu entwickeln, bei dem erheblich höhere Durchsatzleistungen gegenüber konventionellen Verfahren erreichbar sind, insbesondere, wenn ungesättigte Fettsäuremethylester und kurzkettige Fettsäuremethylester im C-Kettenbereich C₆ bis C₁₀ hydriert werden.

Diese Aufgabe wird erfindungsgemäß mit dem Verfahren der eingangs genannten Art dadurch gelöst, daß die Hydrierreaktion in einem über ein Kühl- oder Heizfluid isotherm eingestellten Rohrbündelreaktor durchgeführt wird, wobei die flüssige Phase ohne Rückvermischung neben der gasförmigen Phase als Gleichstromrieselphase über Katalysatorschüttungen in den Reaktoreinzelrohren geleitet wird, und daß die Volumenbelastung des Reaktors zwischen 0,2 und 2,5 Liter Einsatzstoff pro Liter Reaktorvolumen und Stunde und die Flächenbelastung jedes Reaktoreinzelrohres zwischen 1,5 und 24 m³ Einsatzstoff pro m² Reaktorquerschnitt und Stunde gewählt wird und die Reaktionsparameter Temperatur und Druck der aktuellen Katalysatoraktivität entsprechend angepaßt werden. Bei diesem erfindungsgemäßen Verfahren werden bis zu 3fach höhere Durchsatzleistungen gegenüber Verfahren mit konventionellen Reaktoren erreicht. Das bedeutet eine Verringerung des Reaktorvolumens um den gleichen Faktor. Die Reaktionswärme wird größtenteils über die Reaktorwand abgeführt, so daß eine praktisch isotherme Fahrweise möglich ist. Der Katalysator wird dadurch geschont und arbeitet selektiver, und seine Lebensdauer erhöht sich.

Während in den konventionellen Schachtreaktoren zum Erniedrigen der Exothermie und zum Aufrechterhalten günstiger Strömungsverhältnisse hohe Wasserstoffkreisgasmengen erforderlich sind, werden im Rohrbündelreaktor trotz höherer Leistung deutlich niedrigere Kreisgasmengen benötigt. Eine Verringerung des Kreisgasstroms wirkt sich darüber hinaus äußerst günstig auf die Investitionskosten der Anlage aus. Weiterhin wird aufgrund der insgesamt niedrigen Gesamtlänge des Rohrbündelreaktors ein verringerter Druckabfall im Reaktor beobachtet, so daß Kompressionsenergie eingespart wird.

Mit dieser Verfahrensführung kann die Hydrierreaktion so gesteuert werden, daß die Reaktion auf der Stufe der gewünschten Reaktionsprodukte angehalten wird. Diese Reaktionssteuerung wird dadurch erreicht, daß die fluiden Phasen ohne Rückvermischung mit einer definierten Verweilzeit durch die Katalysatorschüttungen in den Reaktoreinzelrohren geführt werden. Dabei werden die Reaktionsparameter Temperatur und Druck entsprechend der jeweiligen Katalysatoraktivität solange aufeinander abgestimmt, bis sich die gewünschten Produktausbeuten einstellen. Die isotherme Temperaturführung im Rohrbündelreaktor gewährleistet, daß nur die gewünschten Reaktionsmechanismen ablaufen.

Aus der DE 37 24 257 A1 ist zwar ein Verfahren zur Hydrierung von Fettsäuremethylestergemischen bekannt, aber die dort angeführten Beispiele betreffen nur Labortests zur Bestimmung der Aktivität des dort beschriebenen Katalysators. Schlüsse auf das hier beschriebene erfindungsgemäße technische Hydrierverfahren lassen sich daraus nicht ziehen.

Vorzugsweise beträgt in dieser Erfindung die spezifische Kreisgasmenge 1200 bis 6500, insbesondere 2000 bis 3000 Nm³/Nm³ Ester h.

In einer vorteilhaften Ausgestaltung sieht die Erfindung vor, daß zum Vermeiden von Entmischungen der Innendurchmesser der Reaktoreinzelrohre zwischen 25 und 400 mm, vorzugsweise 30 und 100 mm, insbesondere 40 bis 70 mm gewählt wird, und die mobilen Phasen in Pfropfstromcharakteristik durch die Katalysatorschüttung geleitet werden. Pfropfstromcharakteristik bedeutet, daß die Strömungsgeschwindigkeiten sowohl der Gas- als auch der Flüssigphase in allen Reaktionsrohren gleich sind und damit eine enge Verweilzeit erreicht wird. Durch diese Ausgestaltung der Reaktoreinzelrohre wird sichergestellt, daß der Katalysator gleichmäßig benetzt ist und somit keine unkontrollierten Reaktionsabläufe auftreten können. So wird eine exakte Reaktionsführung zum Erzielen des gewünschten Reaktionsproduktes gewährleistet.

Besonders zweckmäßig ist es, wenn die Volumenbelastung auf Werte zwischen 0,3 und 2,0 Liter Einsatzstoff pro Liter Reaktorvolumen und Stunde eingestellt wird. Dabei wird die Flächenbelastung jedes Reaktoreinzelrohres vorteilhaft auf Werte zwischen 1,5 und 15 m³ Einsatzstoff pro m² Reaktorquerschnitt und Stunde eingestellt. Mit diesen speziellen Verfahrensbedingungen ist eine besonders exakte Reaktionsführung möglich.

Eine weitere bevorzugte Ausführungsform der Erfindung sieht vor, daß durch interne Kühlung über einen Wasserstoffüberschuß und/oder durch externe Kühlung über das Kühlfluid die maximale Temperaturerhöhung in der Reaktionszone auf Werte bis maximal 5 °C eingestellt wird. Durch diese sehr genaue Temperaturführung ist sichergestellt, daß keine unerwünschten Folgereaktionen auftreten und der Katalysator nicht thermisch geschädigt wird.

Nach einer weiteren Ausgestaltung der Erfindung ist vorgesehen, daß das Verfahren bei Temperaturen bei 180 und 250 °C und bei Drücken zwischen 150 und 280 bar durchgeführt wird. Diese Verfahrensparameter haben sich als besonders günstig erwiesen.

Zweckmäßig ist auch, wenn die Reaktoreinzelrohre über einen Verteiler gleichmäßig mit einer Genauigkeit von 5 % mit flüssiger Phase beaufschlagt werden. Dadurch ist gewährleistet, daß in allen Reaktionsrohren ein einheitlicher Reaktionsablauf stattfindet, so daß ein einheitliches Reaktionsprodukt entsteht.

Ferner wird vorgeschlagen, daß die Reaktoreinzelrohre über einen zweistufigen Flüssigkeitsverteiler gleichmäßig mit flüssiger Phase beaufschlagt werden. Dadurch wird eine besonders günstige enge Verweilzeitverteilung der Gas- und Flüssigphase erreicht.

In einer weiteren Ausgestaltung wird schließlich vorgeschlagen, daß dem Einsatzprodukt kein Methanol zugesetzt wird. In diesem Verfahren ist ein Zusatz von Methanol zum Einsatzstoff nämlich nicht erforderlich. Bedingt durch die direkte Wärmeabführmöglichkeit ist eine schonendere Reduktion des Katalysators und eine erhöhte Sicherheit während des Betriebs gewährleistet.

Im folgenden wird die Erfindung anhand der tabellarisch dargestellten Ausführungsbeispiele erläutert.

| | 1* | 2* | 3 | Vergleichsbeispiel |
|---|---|---|---|---|
| Einsatzstoff | C 12 - C 18 - Fettsäuremethylester (dest.) | | | |
| Reaktor Typ | Rohrreaktor isotherm | Rohrreaktor isotherm | Rohrbündelreakt. isotherm | Schachtreaktor adiabatisch |
| Rohranordnung | - | - | parallel | in Reihe |
| Anzahl Rohre | 1 | 1 | 31 | 2 |
| Länge (m) | 6 | 6 | 6,5 | 7,25 |
| Durchmesser (innen) (mm) | 63 | 63 | 63,5 | 1340 |
| Kata-Typ | CuZn | CuZn | CuZn | CuZn |
| Form | zyl. Tablt. | zyl. Tablt. | zyl. Tabletten | zyl. Tabletten |
| Größe (mm) | 3 x 3 | 4 x 4 | 4 x 4 | 6 x 6 |
| Reakt.druck (bar) | 250 | 250 | 250 | 250 |
| Eintrittstemperatur (°C) | 228 | 224 | 226 | 225 |
| Austrittstemperatur (°C) | 227 | 224 | 226 | 240 |
| Kata.volumen (l) | 16 | 16 | 600 | 17000 |
| Feed-Durchs. (l/h) | 24 | 24 | 1600 | 8000 |
| LHSV (h⁻¹) | 1,5 | 1,5 | 1,5 | 0,4706 |
| Methanoldurchsatz (l/h) | - | - | - | 900 |
| Kreisgas (Druck m³/h) | 1,6 | 0,5 | 15 | 432 |
| Kreisgas (Druck m³/m³ Ester/h) | 66,67 | 20,84 | 9 | 54 |
| | | | | |
| Produktspez. VZ | 1,2 | 1,2 | 1,2 | 1,2 |
| KW (Gew.-%) | 0,3 - 0,5 | 0,3 - 0,5 | 0,3 - 0,5 | 0,5 - 0,7 |
| Kata.abschreibung | < 0,1 % | - | < 0,13 % | 0,15 % |

| | | | | |
|---|---|---|---|---|
| * = zum Vergleich | | | | |

## Patentansprüche

1. Verfahren zur katalytischen Hydrierung von flüssigen gesättigten und ungesättigten Fettsäure-Methylestern im C-Kettenbereich von C₆ bis C₂₄ zur Gewinnung von gesättigten Fettalkoholen und Methanol in Gegenwart von gasförmigem Wasserstoff und Hydrierkatalysatoren bei Drücken von 50 bis 300 bar und Temperaturen von 160 bis 250 °C,
dadurch gekennzeichnet,
daß die Hydrierreaktion in einem über ein Kühl- oder Heizfluid isotherm eingestellten Rohrbündelreaktor durchgeführt wird, wobei die flüssige Phase ohne Rückvermischung neben der gasförmigen Phase als Gleichstromrieselphase über Katalysatorschüttungen in den Reaktoreinzelrohren geleitet wird, und daß die Volumenbelastung des Reaktors zwischen 0,2 und 2,5 l Einsatzstoff pro l Reaktorvolumen und Stunde und die Flächenbelastung jedes Reaktoreinzelrohres zwischen 1,5 und 24 m³ Einsatzstoff pro m² Reaktorquerschnitt und Stunde gewählt wird und die Reaktionsparameter Temperatur und Druck der aktuellen Katalysatoraktivität entsprechend angepaßt werden.

2. Verfahren nach Anspruch 1 oder 2
dadurch gekennzeichnet,
daß die spezifische Kreisgasmenge 1200 bis 6500, insbesondere 2000 bis 3000 Nm³/Nm³ Ester h beträgt.

3. Verfahren nach Anspruch 1 oder 2
dadurch gekennzeichnet,
daß zum Vermeiden von Entmischungen der Innendurchmesser der Reaktoreinzelrohre zwischen 25 und 400 mm, vorzugsweise 30 und 100 mm, insbesondere 40 und 70 mm gewählt wird und die mobilen Phasen in Pfropfstromcharakteristik durch die Katalysatorschüttung geleitet werden.

4. Verfahren nach einem der Ansprüche 1 bis 3,
dadurch gekennzeichnet,
daß die Volumenbelastung auf Werte zwischen 0,3 und 2,0 l Einsatzstoff pro l Reaktorvolumen und Stunde eingestellt wird.

5. Verfahren nach einem der Ansprüche 1 bis 4,
dadurch gekennzeichnet,
daß die Flächenbelastung jedes Reaktoreinzelrohres auf Werte zwischen 1,5 und 15 m³ Einsatzstoff pro m² Reaktorquerschnitt und Stunde eingestellt wird.

6. Verfahren nach einem der Ansprüche 1 bis 5,
dadurch gekennzeichnet,
daß durch interne Kühlung über einen Wasserstoffüberschuß und/oder durch externe Kühlung über das Kühlfluid die maximale Temperaturerhöhung in der Reaktionszone auf Werte bis maximal 5 °C eingestellt wird.

7. Verfahren nach einem der Ansprüche 1 bis 6,
dadurch gekennzeichnet,
daß es bei Temperaturen zwischen 180 und 250 °C durchgeführt wird.

8. Verfahren nach einem der Ansprüche 1 bis 7,
dadurch gekennzeichnet,
daß es bei Drücken zwischen 150 und 280 bar durchgeführt wird.

9. Verfahren nach einem der Ansprüche 1 bis 8,
dadurch gekennzeichnet,
daß die Reaktoreinzelrohre über einen Verteiler gleichmäßig mit einer Genauigkeit von 5 % mit flüssiger Phase beaufschlagt werden.

10. Verfahren nach einem der Ansprüche 1 bis 9,
dadurch gekennzeichnet,
daß die Reaktoreinzelrohre über einen zweistufigen Flüssigkeitsverteiler gleichmäßig mit flüssiger Phase beaufschlagt werden.

11. Verfahren nach einem der Ansprüche 1 bis 10,
dadurch gekennzeichnet,
daß dem Einsatzprodukt kein Methanol zugesetzt wird.

12. Anwendung des Verfahrens nach einem der Ansprüche 1 bis 11 auf Fettsäure-Methylester im C-Kettenbereich von C₆ bis C₁₀.

13. Anwendung des Verfahrens nach einem der Ansprüche 1 bis 11 auf Fettsäure-Methylester im C-Kettenbereich von C₁₂ bis C₁₈.

14. Anwendung des Verfahrens nach einem der Ansprüche 1 bis 11 auf Ester mit hoher Jodzahl.

## Claims

1. A process for the catalytic hydrogenation of liquid saturated and unsaturated c₆₋₂₄ fatty acid methyl esters for the production of saturated fatty alcohols and methanol in the presence of gaseous hydrogen and hydrogenation catalysts under pressures of 50 to 300 bar and at temperatures in the range from 160 to 250°C, characterized in that the hydrogenation reaction is carried out in a tube bundle reactor in which isothermal conditions are established by a cooling or heating fluid, the liquid phase and gas phase being passed together as a co-current trickle phase over catalyst packings in the individual tubes of the reactor without any back-mixing, and in that the load per unit volume of the reactor is between 0.2 and 2.5 liters starting material per liter reactor volume per hour and the load per unit area of each individual tube of the reactor is between 1.5 and 24 m³ starting material per m₂ reactor cross-section per hour and the reaction parameters of temperature and pressure are correspondingly adapted to the particular activity of the catalyst.

2. A process as claimed in claim 1 or 2, characterized in that the specific recycle gas volume is from 1,200 to 6,500 and more particularly from 2,000 to 3,000 Nm³/Nm³ ester/h.

3. A process as claimed in claim 1 or 2, characterized in that, to avoid separation, the internal diameter of the individual tubes of the reactor is selected between 25 and 400 mm, preferably between 30 and 100 mm and more preferably between 40 and 70 mm and the mobile phases are passed through the catalyst packing in plug flow characteristic.

4. A process as claimed in any of claims 1 to 3, characterized in that the load per unit volume is adjusted to values of 0.3 to 2.0 l starting material per liter reactor volume per hour.

5. A process as claimed in any of claims 1 to 4, characterized in that the load per unit area of each individual reactor tube is adjusted to values of 1.5 to 15 m³ starting material per m² reactor cross section per hour.

6. A process as claimed in any of claims 1 to 5, characterized in that the maximum temperature increase in the reaction zone is adjusted to values of at most 5°C by internal cooling through an excess of hydrogen and/or by external cooling through the cooling fluid.

7. A process as claimed in any of claims 1 to 6, characterized in that it is carried out at temperatures of 180 to 250°C.

8. A process as claimed in any of claims 1 to 7, characterized in that it is carried out under presures of 150 to 280 bar.

9. A process as claimed in any of claims 1 to 8, characterized in that the individual reactor tubes are uniformly charged with liquid phase to an accuracy of 5% through a distributor.

10. A process as claimed in any of claims 1 to 9, characterized in that the individual reactor tubes are uniformly charged with liquid phase through a two-stage liquid distributor.

11. A process as claimed in any of claims 1 to 10, characterized in that no methanol is added to the starting product.

12. The application of the process claimed in any of claims 1 to 11 to C₆₋₁₀ fatty acid methyl esters.

13. The application of the process claimed in any of claims 1 to 11 to C₁₂₋₁₈ fatty acid methyl esters.

14. The application of the process claimed in any of claims 1 to 11 to esters of high iodine value.

## Revendications

1. Procédé pour l'hydrogénation catalytique d'esters méthyliques d'acides gras liquides, saturés et non saturés au niveau des chaînes carbonées de C₆ à C₂₄ pour l'obtention d'alcools gras saturés et de méthanol en présence d'hydrogène gazeux et de catalyseurs d'hydrogénation à des pressions de 50 à 300 bars et des températures de 160 à 250°C, caractérisé en ce que la réaction d'hydrogénation est exécutée dans un réacteur à faisceau tubulaire ajusté de manière isothermique sur un liquide de refroidissement ou de chauffage; la phase liquide étant dirigée sans remélangeage à côté de la phase gazeuse comme phase de ruissellement en écoulement continu sur des répartitions de catalyseurs dans les tubes séparés du réacteur, et en ce que la charge volumique du réacteur est choisie entre 0,2 et 2,5 ℓ de charge par volume de réacteur et par heure et les paramètres de la réaction température et pression sont adaptés suivant l'activité actuelle du catalyseur.

2. Procédé selon les revendications 1 ou 2, caractérisé en ce que la quantité spécifique de gaz en circuit atteint 1200 à 6500, en particulier 2000 à 3000 Nm³/Nm³ d'ester par heure.

3. Procédé selon les revendications 1 ou 2, caractérisé en ce que pour éviter des ségrégations le diamètre intérieur des tubes individuels du réacteur est choisi entre 25 et 400 mm, de préférence entre 30 et 100 mm et tout particulièrement entre 40 et 70 mm et les phases mobiles sont guidées en caractéristique d'écoulement en régime tampon à travers la répartition de catalyseur.

4. Procédé selon une des revendications 1 à 3, caractérisé en ce que la charge volumique est ajustée à des valeurs comprises entre 0,3 et 2,0 ℓ de charge par litre de volume du réacteur et par heure.

5. Procédé selon une des revendications 1 à 4, caractérisé en ce que la charge en surface de chaque tube individuel du réacteur est ajustée à des valeurs comprises entre 1,5 et 15 m³ de charge par m² de section transversale du réacteur et par heure

6. Procédé selon une des revendications 1 à 5, caractérisé en ce que grâce au refroidissement interne causé par un excès d'hydrogène et/ou grâce à un refroidissement externe par l'intermédiaire du liquide de refroidissement l'élévation de température maximale dans la zone réactionnelle est ajustée à des valeurs jusqu'à un maximum de 5°C.

7. Procédé selon une des revendications 1 à 6, caractérisé en ce qu'il est réalisé à des températures comprises entre 180 et 250°C.

8. Procédé selon une des revendications 1 à 7, caractérisé en ce qu'il est réalisé à des pressions comprises entre 150 et 280 bars.

9. Procédé selon une des revendications 1 à 8, caractérisé en ce que les tubes individuels du réacteur sont soumis à la phase liquide par l'intermédiaire d'un répartiteur de manière homogène avec une précision de 5 %.

10. Procédé selon une des revendications 1 à 9, caractérisé en ce que les tubes individuels du réacteur sont soumis à la phase liquide par l'intermédiaire d'un répartiteur de liquide à deux étages d'une manière homogène.

11. Procédé selon une des revendications 1 à 10, caractérisé en ce qu'au produit constituant la charge on n'ajoute pas de méthanol.

12. Utilisation du procédé selon une des revendications 1 à 11, pour des esters méthyliques d'acides gras au niveau de chaînes carbonées de C₆ à C₁₀.

13. Utilisation du procédé selon une des revendications 1 à 11, pour des esters méthyliques d'acides gras au niveau de chaînes carbonées de C₁₂ à C₁₈.

14. Utilisation du procédé selon une des revendications 1 à 11 sur des esters à indice d'iode élevé.
